# EUROPEAN PATENT APPLICATION

(11) **EP 2 033 957 A1**
(43) Date of publication of application: **11.03.2009**
(21) Application number: 07115782.0
(22) Date of filing: 06.09.2007
(51) Int. Cl.: C07D 263/22, C07F 1/08

(54) **Process for the manufacture of alkyl compounds**

(71) Applicant: SOLVAY (Société Anonyme), 1050 Bruxelles (BE)
(72) Inventor: Pousset, Cyrille, 1070, BRUSSELS (BE); Callens, Roland, 1850, GRIMBERGEN (BE); Gire, Ronan, 1050, BRUSSELS (BE)
(74) Representative: Mross, Stefan P.M.

(57) **Abstract**

Process for the manufacture of a compound of formula I 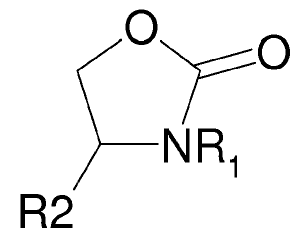 wherein R1 is H, acyl, alkyl or carbamoyl, R2 is a linear alkyl or alkenyl group, preferably a linear alkyl group comprising from 6 to 20 carbon atoms and n is 0, 1, 2, or 3, which comprises reacting a compound of formula II 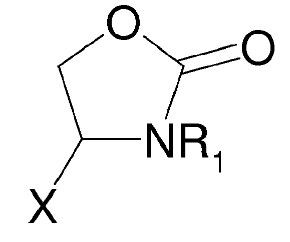 wherein R1 and n are as above and X is a leaving group with a carbanionic reagent of formula II

M-R2

wherein R2 is as above and M denotes a metal or a metal containing group.

## Description

The present invention relates to a process for the manufacture of long chain alkyl compounds and to reagents for use in such manufacture.

Long-chain alkyl compounds are useful, amongst others in pharmaceutical or in particular cosmetic preparations, where they act as skin penetration enhancers. A particular advantageous compound of this class is 4-decyloxazolidin-2-one which is commercialized by Girindus AG.

WO-A-90/00407 discloses the manufacture of the latter compound by reaction of 2-aminododecan-1-ol with ethylene carbonate. However, the 2-aminododecan-1-ol required is not readily available.

According to Ishizuka et al., Chem. Lett. 1992, p.991-994, certain chiral 4-substituted 2-oxazolidinones have been manufactured by a reaction sequence involving addition of certain organometallics, including organocuprates, to 4-methoxy-2-oxazolidinone. However that reference is unrelated to the manufacture of long chain alkyl compounds.

Now, applicants have found a process which allows for efficient and selective manufacture of long chain alkyl compounds such as in particular 4-decyloxazolidin-2-one.

The invention concerns in consequence a process for the manufacture of a compound of formula I 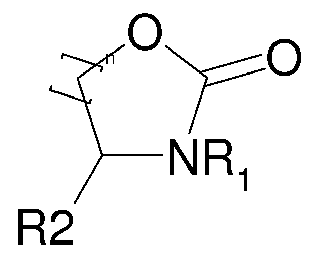 wherein R1 is H, acyl, alkyl or carbamoyl, R2 is a linear alkyl or alkenyl group, preferably a linear alkyl group comprising from 6 to 20 carbon atoms and n is 0, 1, 2, or 3, which comprises reacting a compound of formula II 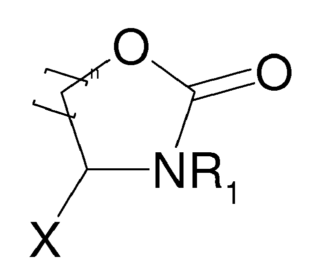 wherein R1 and n are as above and X is a leaving group with a reagent of formula III

M-R2

wherein R2 is as above and M denotes a metal or a metal containing group.

The process according to the invention allows in particular for efficient industrializable production of long chain alkyl substituted compounds. This is unexpected, as reactivity of long-chain organometallics is expected to decrease.

Compounds of formula II are readily available for example starting from commercially available oxazolidin-2-one.

An embodiment of the process according to the invention consequently further comprises synthesizing the compound of formula II by electrochemically oxidizing a compound of formula IV 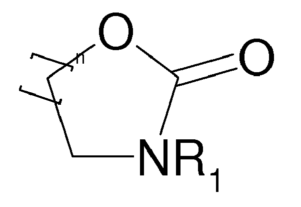 wherein R1 is H, acyl, alkyl or carbamoyl and n is 0, 1, 2, or 3, in the presence of a precursor of leaving group X.

Electrochemical oxidation of oxazolidin-2-one to provide certain 4-substituted oxazolidin-2-ones was described in Tavernier et al. Bull. Soc. Chim. Belg. 1988, p.859-865, however this reference falls short of any suggestion that the 4-substituted oxazolidin-2-ones disclosed therein could be reactive in the present invention and be efficiently converted into oxazolidin-2-ones having a long-chain alkyl substituent in 4-position.

In the process according to the invention, the leaving group X is often a substituent which is bonded to the heterocycle through a sulphur or, preferably, an oxygen atom. Examples of O-bonded leaving groups are alkoxy or acyloxy groups. Alkoxy groups useful as substitutent X generally contain 1 to 4 carbon atoms. Ethoxy and, in particular, methoxy are preferred. Acyloxy groups useful as substitutent X generally contain 2 to 10 carbon atoms. Propionyloxy, and, in particular, acetoxy are preferred.

In the process according to the invention, if the substituent R1 is a linear alkyl group it is often a C1 to C10 alkyl group, more specifically a methyl or an ethyl group. If the substituent R1 is an acyl group it is often a C1 to C10 acyl group, more specifically a formyl or, preferably, an acetyl group. In a particularly preferred embodiment, R1 is H.

The process according to the invention applies in particular to compounds wherein n is 1 or 2, preferably 1. It is understood that the process according to the invention will equally apply to compounds in which the optional methylene group or groups shown in the formulae bear one or more substituents which are substantially stable under the conditions of the reaction. Such substituents are for example alkyl or aryl substituents. However the process according to the invention applies preferably when all groups are methylene. It applies more particularly to the manufacture of 4-n-decyloxazolidin-2-one.

In the process according to the invention, the substituent R2 is generally a linear C6-C20 alkyl group. R2 is preferably a linear alkyl group comprising 8, 9, 10, 11, 12, 13, 14, 15 or 16 carbon atoms and more preferably 8, 10, 12, 14 or 16 carbon atoms. An n-decyl group is more particularly preferred as R2.

In the process according to the invention M in the reagent M-R2 contains often a metal selected from lithium, magnesium, or transition metals, such as copper, zinc, cadmium, mercury, cerium and zirconium. Often the metal is selected from copper, zinc, cadmium, mercury, cerium and zirconium and is preferably copper.

The reagents M-R2 can suitably be obtained either through direct reaction or by transmetallation from the corresponding halides Y-R2 wherein Y is selected from Cl, Br and I. It is preferred to use a bromide Br-R2 as starting material.

In a particular embodiment, which is particularly preferred when the leaving group X is an O-bonded leaving group as described herein before, M contains copper. A particularly preferred reagent has the formula Cu-R2 wherein R2 is as described above.

A suitable copper containing reagent can be obtained, for example, by the reaction of a Grignard reagent as described above with a copper precursor. Suitable copper precursors are selected for example from copper (I) complexes or salts such as CuBr, CuI, CuCN or complexes of these salts with thioethers such as in particular dimethylsulphide.

In the process according to the invention, the reagent can be suitably activated with an activator such as for example a Lewis acid. Boron compounds such as in particular BF₃ which can be supplied to the reaction as adduct with an ether such as diethylether can be used, in particular when M contains copper. In another aspect the activator is an active silyl compound such as a halotrialkylsilane in particular trimethylchlorosilane. A particularly preferred activated reagent corresponds in consequence to the formula BF₃.Cu-R2.

The invention also concerns the copper-containing reagents described here before. It has been found that these reagents are particularly efficient to introduce long-chain alkyl residues by nucleophilic substitution notably of O-bonded leaving groups as described herein before.

In the process according to the invention, the reaction is generally carried out in an organic solvent. This solvent is generally an inert more specifically an aprotic solvent, what means that the solvent does not interfere with the reaction of the compound of formula II with the reagent. Examples of suitable solvents include alkylethers such as diethylether, methyl-tert.butylether, tetrahydrofurane and 1,4-dioxane. Tetrahydrofurane is preferred as solvent.

The invention also concerns a solution of the copper-containing reagents described here before in the solvents described here before.

The invention concerns also the use of the copper-containing reagent or its solution for the manufacture of an organic compound containing a C6-C20 linear alkyl substitutent. In the use according to the invention, the manufacture of the organic compound is preferably carried out by nucleophilic substitution of an O-bonded leaving group as described herein before. More preferably, the O-bonded leaving group is attached to a carbon atom in α-position to a nitrogen atom. Said nitrogen atom is preferably an optionally alkylated or acylated amine.

Particular examples of compounds containing an O-bonded leaving group is attached to a carbon atom in α-position to a nitrogen atom correspond to the formula IV 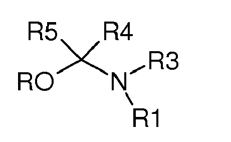 wherein R1 is as described above, RO is an O-bonded leaving group as described above, preferably methoxy, R3 and R4 are independently selected from H, and optionally substituted alkyl, aryl or aralkyl groups or preferably R3 and R4 form together a heterocycle which contains the nitrogen atom shown in the formula, which heterocycle is preferably 4- to 7-membered and more preferably 5- or 6-membered and which can optionally contain further heteroatoms in the ring and R5 is a substituent, preferably H.

In the process according to the invention or the use according to the invention the reaction is generally carried out at a temperature from -80°C to 50°C, preferably from -50°C to 30°C and more preferably from -40°C to 25°C.

In the process according to the invention or the use according to the invention the molar ratio of the reagent M-R2 to the compound of formula II or IV respectively is generally equal to or greater than 1, preferably equal to or greater than about 1.2. Generally this molar ratio is lower than about 4, often equal to or lower than 3, preferably equal to or lower than 2.

It has been found, surprisingly, that high yield of long chain alkyl compound can be obtained with a moderate quantity of organometallic reagent thus allowing for more economic manufacturing.

In general, the reaction is carried out in the substantial absence of water. Typical contents of water in the reaction medium are from 1 mg/kg to 1000 mg/kg relative to the total weight of the reaction medium. Preferably the water contents is from 5 mg/kg to 100 mg/kg.

The reaction mixture can be worked up by extraction/crystallization. It has been found that notably the isolation of 4-decyloxazolidin-2-one can be improved by (a) controlling and optionally reducing the water content of a crude product obtained from extraction and recrystallizing the dry crude product.

When the process according to the invention further comprises synthesizing the compound of formula II by electrochemically oxidizing a compound of formula IV, the precursor of leaving group X is advantageously selected from methanol, acetic anhydride and acetic acid. In a particular preferred embodiment the precursor of leaving group X consists essentially of methanol.

In this embodiment, the electrochemical oxidation can be carried out in a compartmentalized or non-compartmentalized cell.

The electrodes used in the electrochemical oxidation should be resistant with respect to the conditions of the electrochemical reaction. Suitable materials are in particular chosen from metals, metal oxides and graphite. Particularly suitable metals are chosen from steel, iron and titanium, and in particular from the metals of the group of platinum and their oxides, or electrodes coated with the latter materials. Platinum or rhodium is preferred. An electrode comprising platinum is particularly suitable.

The distance between the electrodes is generally at least 0.2 mm. This distance is often at least 0.5 mm. It is preferably at least 1 mm. The distance between the electrodes is generally at most 20 mm. This distance is often at most 10 mm. It is preferably at most 5 mm.

In this embodiment, the electrochemical oxidation is generally carried out at a current density greater than or equal to 0.1 A/dm². The current density is often greater than or equal to 1 A/dm². It is preferably greater than or equal to 3 A/dm². In this embodiment, the electrochemical oxidation is generally carried out at a current density less than or equal to 50 A/dm². The current density is often less than or equal to 30 A/dm². It is preferably less than or equal to 20 A/dm².

In this embodiment, the electrochemical oxidation is generally carried out at a temperature greater than or equal to -50°C. The temperature is often greater than or equal to -20°C. It is preferably greater than or equal to 0°C. In this embodiment, the electrochemical oxidation is generally carried out at a temperature less than or equal to 100°C. The temperature is often less than or equal to 80°C. It is preferably less than or equal to 60°C.

In this embodiment, the electrochemical oxidation is generally carried out in the presence of a conducting salt such as tetraalkyl ammonium tetrafluoroborate in particular tetrabutylammonium tetrafluoroborate, alkali tetrafluoroborate such as potassium tetrafluoroborate or alkali perchlorates, preferably lithium perchlorate. It has been found that alkali perchlorates, in particular lithium perchlorate allow for highly selective manufacture of compound of formula II and allow for efficient isolation of compound of formula II from the reaction medium.

The examples below are intended to illustrate the invention without, however, limiting it.

### Example 1

A solution of the Grignard reagent (1.3 equivalents) obtained by reaction of n-decylbromide with magnesium was added to a stirred suspension of CuBr.Me₂S (1.5 equivalents) in dry THF, at- 45°C. Subsequently, BF₃.Et₂O (1.5 equivalents) was added at - 50°C and then a solution of 4-methoxy-oxazolidin-2-one (1 equivalent) in THF was added. The mixture was stirred and allowed to reach room temperature. After 4 h the conversion was 100 %, the reaction was quenched with a mixture of an aqueous saturated NH₄Cl solution and diluted NH₄OH 28 %. The product 4-n-decyloxazolidin-2-one was isolated by extraction. The crude product was dried by azeotropic distillation of water with ethyl acetate. This allowed for improved solidification/crystallization.

Yield 68 % purity GC > 99 %.

### Example 2

To a THF (50cm³) solution of cuprous cyanide (1.99g, 22 mmol) and anhydrous lithium chloride (1.87 g, 44 mmol), n-decylmagnesium bromide (20 cm³, 1 M in THF) was dropwise added at - 30°C under nitrogen atmosphere. 4-methoxy-2-oxazolidin-2-one (0.85 g, 5 mmol) and BF₃.Et₂O (1.27 cm³, 10 mmol) were added successively and the reaction mixture was kept at - 30°C overnight. The reaction was quenched by addition of saturated NH₄Cl solution (5 cm³) and extracted twice with Et₂O (100 cm³). Organic phases were concentrated in vacuo and the residue was precipated at low temperature to give an isolated yield of 4-n-decyloxazolidin-2-one of 0.54 g, (48 %).

### Example 3

Oxazolidin-2-one was dissolved in acetic acid, and added to dry methanol containing tetrabutylammoniumtetrafluoroborate, in an undivided dry cell equipped with a platinum anode at 10°C and an electric current of 7A for 9 hours.

After partial evaporation of the solvents, crystallization of the residue at 0°C yielded 4-methoxy-oxazolidin-2-one.

Yield 61 % purity GC 99 %.

### Example 4

Oxazolidin-2-on (355.5 g, 4 moles) dissolved in methanol (1100 cm³) containing lithium perchlorate (7.74 g, 72 mmoles), was subjected to methoxylation in an undivided dry cell equipped with a platinum anode at 18°C and an electric current of 7 A for 41.4 hours. Consumption of oxazolidin-2-on was monitored by GC. After evaporation of the solvents, crystallization of the residue in isopropyl acetate and tert-butlymethyl ether yielded 4-methoxy-oxazolidin-2-on Yield 74 % purity GC 98 %.

## Claims

1. Process for the manufacture of a compound of formula I 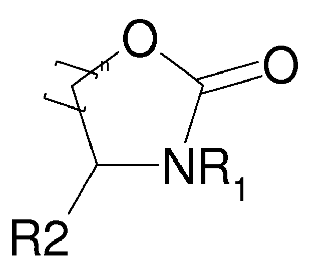 wherein R1 is H, acyl, alkyl or carbamoyl, R2 is a linear alkyl or alkenyl group, preferably a linear alkyl group, comprising from 6 to 20 carbon atoms and n is 0, 1, 2, or 3, which comprises reacting a compound of formula II 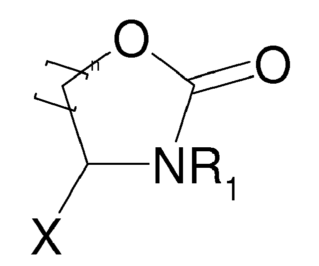 wherein R1 and n are as above and X is a leaving group with a reagent of formula III
M-R2
wherein R2 is as above and M denotes a metal or a metal containing group.

2. Process according to claim 1 wherein X is an alkoxy or an acyloxy group.

3. Process according to claim 2, wherein X is a methoxy group.

4. Process according to claim 2, wherein X is an acetyl group.

5. Process according to anyone of claims 1 to 4, wherein R1 is H.

6. Process according to anyone of claims 1 to 5 wherein n is 1.

7. Process according to anyone of claims 1 to 6 wherein R2 is n-decyl.

8. Process according to anyone of claims 1 to 7 wherein M contains copper.

9. Process according to claim 8 wherein M is Cu.BF₃.

10. Process anyone of claims 1 to 9 wherein the molar ratio of reagent M-R2 to compound of formula II is from 1 to less than 4.

11. Process according to claim 10, wherein the molar ratio is from 1.2 to 2.

12. Process according to anyone of claims 1 to 11 wherein the reaction is carried out at a temperature from -80°C to +50°C.

13. Process according to anyone of claims 1 to 12 wherein the reaction is carried out in an organic aprotic solvent.

14. Process according to anyone of claims 1 to 13 which further comprises synthetizing the compound of formula II by electrochemically oxidizing a compound of formula IV 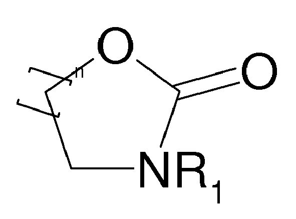 wherein R1 is H, acyl, alkyl or carbamoyl and n is 0, 1, 2, or 3 in the presence of a precursor of leaving group X.

15. Process according to claim 14 wherein the precursor of leaving group X is selected from methanol, acetic anhydride and acetic acid.

16. Process according to claim 15 wherein the precursor of leaving group X consists essentially of methanol.

17. Process according to anyone of claims 14 to 16, wherein the electrochemical oxidation is carried out in the presence of lithium perchlorate as conducting salt.

18. A reagent of formula M-R2 wherein M contains copper and R2 is a C6-C20 linear alkyl residue.

19. The reagent of claim 18, wherein R2 is n-decyl.

20. Use of the reagent according to claims 19 or 20 for the manufacture of an organic compound containing a linear C6-C20 alkyl substituent.
